# EUROPEAN PATENT APPLICATION

(11) **EP 4 567 128 A1**
(43) Date of publication of application: **11.06.2025**
(21) Application number: 23215037.5
(22) Date of filing: 07.12.2023
(51) Int. Cl.: C12Q 1/6874

(54) **IMPROVED METHOD AND MEANS FOR SPATIAL NUCLEIC ACID DETECTION IN-SITU**

(71) Applicant: Max-Delbrück-Centrum für Molekulare Medizin in der Helmholtz-Gemeinschaft, 13125 Berlin (DE); Sapienza University of Rome, 00185 Roma (IT)
(72) Inventor: Rajewsky, Nikolaus, 10707 Berlin (DE); Macino, Guiseppe, 00060 Torrita Tiberina (IT); Karaiskos, Nikolaos, 10961 Berlin (DE); Schott, Lena Marie, 10551 Berlin (DE); Splendiani, Elena, 00157 Rome (IT); Licha, Jan, 14052 Berlin (DE); Ferretti, Elisabetta, 00185 Rome (IT)
(74) Representative: Kuttenkeuler, David

(57) **Abstract**

The invention pertains to a method of spatially resolved detection of nucleic acids in tissue samples. The method of the invention comprises a use of a patterned nanowell structure as a substrate to conduct a transcriptome analysis using clusters of spatially separated capture probes. The invention further provides a method for spatial detection of RNA in a tissue sample, and kits for use in the inventive methods.

## Description

### FIELD OF THE INVENTION

The invention pertains to a method of spatially resolved detection of nucleic acids in tissue samples. The method of the invention comprises a use of a patterned nanowell structure as a substrate to conduct a transcriptome analysis using clusters of spatially separated capture probes. The invention further provides a method for spatial detection of RNA in a tissue sample, and kits for use in the inventive methods.

### DESCRIPTION

Lately, there have been numerous advancements in the field of spatially resolved transcriptome profiling (SRT). The most established and commonly employed SRT techniques entail the hybridization of mRNA onto DNA oligonucleotide probes that incorporate spatial barcodes and unique molecular identifier (UMI) sequences. The captured mRNA is subsequently subjected to reverse transcription (RT), with the capture probe serving as a primer to initiate the RT reaction. The outcome is a cDNA library, where each cDNA molecule contains a spatial barcode, UMI, and mRNA-derived sequence. Given that the spatial barcode can be linked to a specific spatial coordinate and the UMI encodes unique capture events, these methods offer spatial resolution and quantitative capabilities. Notable examples of such techniques include "Spatial Transcriptomics," 10X Genomics Visium, Seq-Scope, STEREOseq, and PIXELseq.

Currently available ST methods are limited by their high costs or difficulty of implementation (academic methods). Additionally, probe-based methods are characterized by biased and species-specific RNA capture limiting the amount of information generated from a single experiment (GeoMx^{®} Digital Spatial Profiler (Nanostring, Merritt et al., 2020), CosMx^{™} Spatial Molecular Imager (Nanostring, He et al., 2022, bioRxiv), Molecular Cartography^{™} (Resolve Biosciences), Visium Spatial Gene Expression for FFPE and Xenium In Situ (10 X genomics, Janesick et al., 2022, bioRxiv)), while poly-dT primed capture approaches in most cases present low spatial resolution (Visium Spatial Gene Expression (10 X genomics)).

Thus, it is an object of the invention to provide improved and optimized methods and means for in situ spatially resolved detection of nucleic acids.

### BRIEF DESCRIPTION OF THE INVENTION

Generally, and by way of brief description, the main aspects of the present invention can be described as follows:

In **a first aspect**, the invention pertains to a method of spatial transcriptomics on a substrate, the method comprising the steps of:
(a) providing a substrate, wherein the substrate comprises at least two spatially separated capture probes immobilized thereon, and wherein the sequence of each of the at least two spatially separated capture probes are associated with its spatial localization on the substrate (spatial barcode);
(b) contacting the substrate with a tissue sample and allowing RNA molecules in the tissue sample to bind to the capture probes;
(c) optionally, imaging the tissue sample while the sample is bound to the substrate;
(d) generating cDNA molecules from the RNA molecules bound to the capture probes;
(e) determining the sequence of the spatial barcode for the cDNA molecules and correlating this sequence with the location of a corresponding cluster on the substrate (e.g., cluster of capture probes containing the corresponding spatial barcode);
*characterized in that,*
the substrate comprises a patterned nanowell array.

In **a second aspect**, the invention pertains to a method for spatial detection of RNA in a tissue sample, comprising:
(a) providing the substrate of any of the preceding claims;
(b) contacting the substrate with a tissue sample;
(c) fixating and permeabilizing the tissue sample and thereby allowing RNA molecules of the fixed and permeabilized tissue sample to bind to the capture domain of the capture probes,
(d) generating cDNA molecules from the bound RNA molecules; and
(e) sequencing the cDNA molecules.

In **a third aspect**, the invention pertains to a kit of parts for use in the spatial transcriptomics, wherein the kit comprises at least a substrate as recited in any one of the preceding claims, and one or more reagents and / or buffers, such as a fixating reagent and a permeabilization reagent.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

Although any methods and materials similar or equivalent to those described herein can be used in the practice or testing of embodiments described herein, some preferred methods, compositions, devices, and materials are described herein. However, before the present materials and methods are described, it is to be understood that this invention is not limited to the particular molecules, compositions, methodologies or protocols herein described, as these may vary in accordance with routine experimentation and optimization. It is also to be understood that the terminology used in the description is for the purpose of describing the particular versions or embodiments only, and is not intended to limit the scope of the embodiments described herein.

Unless otherwise defined, all technical and scientific terms used herein have the same meaning as commonly understood by one of ordinary skill in the art to which this invention belongs. However, in case of conflict, the present specification, including definitions, will control. Accordingly, in the context of the embodiments described herein, the following definitions apply.

The term "substrate" is used herein it the broadest sense and refers to any substrate described herein. The "substrate" may also be referred to herein as a "flow cell surface". The substrate may be a part of a flow cell, wherein the flow cell comprises the flow cell surface (e.g. substrate) and one or more channels (or lanes) to facilitate adding liquids to the flow cell surface. In some embodiments, one or more components of the flow cell are detachable, such that an exposed flow cell surface (e.g. substrate) may be obtained without damaging the capture oligos contained thereupon. In some embodiments, the term "substrate" refers to a substrate generated by methods described herein, such as bridge amplification. In some embodiments, the term "substrate" refers to a second substrate or a replicate substrate formed using an original substrate as a template, and copying the original substrate onto a second media. Methods for spatial detection of nucleic acid in a tissue sample as described herein may be performed using any substrate, including an original substrate and a second substrate.

In **a general aspect**, the invention pertains to a method of spatial detection of biological molecules on a substrate, the method comprising the steps of:
(a) providing a substrate, wherein the substrate comprises at least two spatially separated capture probes immobilized thereon, and wherein the sequence of each of the at least two spatially separated capture probes are associated with its spatial localization on the substrate (spatial barcode);
(b) contacting the substrate with a tissue sample and allowing biological molecules in the tissue sample to bind to the capture probes;
(c) optionally, imaging the tissue sample while the sample is bound to the substrate;
(d) detecting the biological molecules bound to the capture probes and assigning the spatial barcode to the detected biological molecules;
*characterized in that,*
the substrate comprises a patterned nanowell array.

Biological molecules in accordance with this general aspect may be for example proteins or peptides, nucleic acids, such as RNA, DNA or variants thereof, fatty acids, carbohydrates or other biological molecules to be detected.

In specific aspects of the invention, the nucleic acids such as mRNA is detected as biological molecules.

In **a first aspect**, the invention pertains to a method of spatial transcriptomics on a substrate, the method comprising the steps of:
(a) providing a substrate, wherein the substrate comprises at least two spatially separated capture probes immobilized thereon, and wherein the sequence of each of the at least two spatially separated capture probes are associated with its spatial localization on the substrate (spatial barcode);
(b) contacting the substrate with a tissue sample and allowing RNA molecules in the tissue sample to bind to the capture probes;
(c) optionally, imaging the tissue sample while the sample is bound to the substrate;
(d) generating cDNA molecules from the RNA molecules bound to the capture probes;
(e) determining the sequence of the spatial barcode for the cDNA molecules and correlating this sequence with the location of a corresponding cluster on the substrate (e.g., cluster of capture probes containing the corresponding spatial barcode);
*characterized in that,*
the substrate comprises a patterned nanowell array.

In a first preferred embodiment of the invention, step (c) of the method of the first aspect is not optional.

The invention pertains to an improved method for spatial detection of nucleic acids based on the "seq-scope" method. The method is described in WO/2022/015913 which is in incorporated herein in its entirety. However, certain aspects and descriptions of the method are disclosed herein for ease of understanding of the principles of the improved method of the invention. The invention applies the sequencing by synthesis method to generate within a flow cell spatially coded capture probe spots each spot comprising a multiplicity of clonal capture probes which allow attachment of nucleic acids such as poly adenylated nucleic acids (mRNA). An exposed (or opened, preferably using a cutting guide tool of the invention) flow cell with such spots generated thereupon is then used to place thereon a cellular or tissue sample to allow its mRNA molecules to attach to the capture probes of the spots. Using the spatial coding of the spots and a reference image (e.g. optical image) of the cells or tissue on the substrate allows a spatial identification of transcript expression in accordance with the invention.

In context of the invention, in some embodiments, a substrate comprises a plurality of capture probes (e.g. "seeds" or "seed molecules") immobilized on a surface of the substrate. The probes may be immobilized on the surface of the substrate by any suitable means. In some embodiments, the surface of the substrate comprises binding partners for the capture probes. Binding partners for the capture probes are referred to herein as "surface probes". For example, the surface of the substrate may comprise a plurality of surface probes that bind to a complementary adapter region on the capture probe. In some embodiments, the surface of the substrate comprises multiple types of surface probes. For example, the surface of the substrate may comprise two types of surface probes, where the first type of surface probe is complementary to a first adapter region at the 3' end of the capture probe, and the second type of surface probe is complementary to a second adapter region at the 5' end of the capture probe. In such embodiments, clusters of capture probes may be generated on the surface of the substrate by a process known as bridge amplification.

In bridge amplification, the first adapter region at the 3' end of a capture probe binds to the complementary surface probe (e.g., the first type of surface probe). A polymerase enzyme creates a complementary strand to the hybridized capture probe, generating a double stranded molecule. The double stranded molecule is denatured (e.g., by addition of a denaturing agent, such as sodium hydroxide). One or more wash steps may be performed to wash away the original capture probe, leaving behind the complementary strand which is immobilized on the surface of the substrate. By random interaction, the second adapter region at the 3' end of the strand binds to the reverse complementary surface probe (e.g., the second type of surface probe), thus causing the strand to bend, creating a "bridge". Polymerase enzymes generates the complementary strand, creating a double stranded bridge. The double stranded bridge is denatured, resulting in two capture probe(s) having a 5' end bound to the first type of surface probe and an exposed 3' end, one bound to a first adaptor type surface probe and one to a second type adaptor surface probe.

As described above, each capture probe may comprise an adapter region that binds to a complementary surface probe. In some embodiments, each capture probe comprises a capture domain. The capture domain may be any suitable domain capable of hybridizing to a nucleic acid or an RNA transcript thereof, such as mRNA. In some embodiments, the capture domain comprises a poly- T oligonucleotide. A poly-T oligonucleotide comprises a series of consecutive deoxythymidine residues linked by phosphodiester bonds. A poly-T oligonucleotide is capable of hybridizing to the poly-A tail of mRNA. In some embodiments, the capture domain comprises a poly-T oligonucleotide comprising at least 10 deoxythymidine residues. The poly-T oligonucleotide may comprise at least 10, at least 11, at least 12, at least 13, at least 14, at least 15, at least 16, at least 17, at least 18, at least 19, at least 20, at least 25, at least 30, or more than 30 deoxythymidine residues. In some embodiments, the capture domain comprises nucleotides which are functionally or structurally analogous to poly-T and retain the functional property of binding to poly-A. For example, the capture domain may comprise a poly-U oligonucleotide.

In some embodiments, the capture domain is nonspecific (e.g., intended to capture all RNAs containing a poly-A tail). In some embodiments, the capture domain may further comprise additional sequences, such as random sequences, to facilitate the capture of specific subtypes of RNA. In some embodiments, the capture domain may further comprise additional sequences to capture a desired subtype of RNA, such as mRNA, non coding RNA, tRNA or rRNA. In some embodiments, the capture domain may further comprise additional sequences to facilitate the capture of a particular RNA (e.g., mRNA) corresponding to select genes or groups of genes. Such a capture probe may be selected or designed based on sequence of the RNA it is desired to capture. Accordingly, the capture probe may be a sequence-specific capture probe. In some embodiments, the capture domain may target DNA, instead of RNA. In some embodiments, the capture domain may target non-specific or specific DNA sequences. For example, the capture domain may comprise a nucleic acid sequence to facilitate the capture of a target DNA sequence.

In some embodiments, the capture domain for each probe is the same. In some embodiments, the capture domain for one or more probes is different from the capture domain from at least one other probe.

In some embodiments, the capture probes additionally comprise a cleavage domain. In some embodiments, the cleavage domain is 3' of the capture domain, such that the capture domain is not exposed until the cleavage domain is cleaved. For example, the cleavage domain may comprise a binding site (e.g., a restriction site) for a restriction endonuclease. The cleavage domain may be intact (e.g., un-cleaved) during binding of the capture probes to the surface of the substrate and cluster generation. Following cluster generation and/or determination of the location of each cluster on the substrate (e.g., by sequencing of the spatial barcode), an enzyme may be added to induce cleavage of the cleavage domain. For example, a restriction endonuclease (e.g., Xbal, Dral, etc.) may be added to cut the cleavage domain and one or more wash steps may optionally be performed, thus exposing the capture domain.

In some embodiments, cleavage of the cleavage domain may allow for exposure of additional domain(s). For example, cleavage of the cleavage domain may expose the capture domain.

The capture probe comprises a spatial barcode. The spatial barcode may be an oligonucleotide of any suitable length. In some embodiments, the spatial barcode comprises 10-50 nucleotides. For example, the spatial barcode may comprise 10, 11, 12, 13, 14, 15, 16, 17, 18,19, 20, 21, 22, 23, 24, 25, 26, 27, 28, 29, 30, 31, 31, 32, 33, 34, 35, 36, 37, 38, 39, 40, 41, 42, 43, 44, 45, 46, 47, 48, 49, or 50 nucleotides. In particular embodiments, the spatial barcode comprises about 30 nucleotides.

In some embodiments, each capture probe comprises one or more sequencing barcodes (e.g., sequencing handles). For example, each capture probe may comprise a sequencing handle, such as an ILLUMINA TruSeq handle. The sequencing barcode may comprise any suitable number of consecutive nucleotides. In some embodiments, the sequencing barcode comprises 10-50 nucleotides. For example, the sequencing barcode may be about 10, 15, 20, 25, 30, 35, 40, 45, or 50 nucleotides in length.

In some embodiments, each capture probe further comprises one or more filler sequences. The filler sequence may comprise any suitable number of consecutive nucleotides. In some embodiments, the filler sequence comprises 10-50 nucleotides. For example, the filler sequence may be about 10, 15, 20, 25, 30, 35, 40, or 50 nucleotides in length.

The plurality of capture probes is arranged in clusters on the surface of the substrate, each cluster comprising multiple capture probes. Each capture probe in a cluster comprises the same spatial barcode. Additionally, the spatial barcode for each cluster is unique. For example, cluster A contains probes having spatial barcode A, cluster B contains probes having spatial barcode B, cluster C contains probes having spatial barcode C, etc.

In some embodiments, each capture probe in a cluster is engineered to comprise a unique molecular identifier (UMI) (also referred to herein as a "unique molecular identifier barcode" or a "UMI barcode"). Each capture probe in a cluster comprises different UMI barcode (UMI Array). In some embodiments, UMI is not encoded by the capture probe, and instead obtained from the random priming site during secondary strand synthesis. For example, each cDNA will be paired with a secondary strand each of which is encoded by a unique random primer sequence, which is used as UMI (UMI Randomer). UMI Array and UMI Randomer are both efficient in collapsing PCR duplicates from an amplified cDNA library. For example, the sequence of the spatial barcode for each cluster may be determined by next generation sequencing, and duplicate sequence reads may be collapsed through either the unique molecular identifier encoded by the array (UMI Array) or by the random priming site (UMI Randomer). In some embodiments, UMI Randomer may be semi-random so that it has certain nucleotide patterns to make the secondary strand synthesis more efficient.

In some embodiments, each cluster comprises at least 200 capture probes. For example, each cluster may comprise at least 200, at least 300, at least 400, at least 500, at least 600, at least 700, at least 800, at least 900, or at least 1000 capture probes. In some embodiments, each cluster comprises 900-1100 capture probes. For example, each cluster may comprise 900, 910, 920, 930, 940, 950, 960, 970, 980, 990, 1000, 1010, 1020, 1030, 1040, 1050, 1060, 1070, 1080, 1090, or 1100 capture probes. In some embodiments, all capture probes in each cluster will be identical.

Each cluster may be roughly circular in shape. Each cluster may have an average diameter of about 200-1200nm. For example, each cluster may be roughly circular in shape with an average diameter of 200nm, 250nm, 300nm, 350nm, 400nm, 450nm, 500nm, 550nm, 600nm, 650nm, 700nm, 750nm, 800nm, 850nm, 900nm, 950nm, 1000nm, 1050nm, 1100nm, or 1200nm. In some embodiments, each cluster is roughly circular in shape with an average diameter of 950-1050nm. For example, the average diameter may be 950nm, 960nm, 970nm, 980nm, 990nm, I000nm, 1010nm, 1020nm, 1030nm, 1040nm, or 1050nm. In particular embodiments, the average diameter is 550nm (0.55 microns).

The surface of the substrate may comprise any suitable number of clusters. In some embodiments, the surface of the substrate comprises 0.3-2 million clusters per 1mm² of surface. In some embodiments, the surface of the substrate comprises 0.8-1.2 million clusters per 1mm² of surface. In some embodiments, the surface of the substrate comprises about 1 million clusters per 1mm² of surface.

The surface of the substrate may comprise any suitable material. In some embodiments, the surface of the substrate is porous. In some embodiments, the surface of the substrate is nonporous. In some embodiments, the surface comprises a material selected from glass, silicon, poly-L-lysine coated materials, nitrocellulose, polystyrene, polyacrylamide, cyclic olefin copolymers (COCs), cyclic olefin polymers (COPs), polypropylene, polyethylene and polycarbonate. In some embodiments, the surface comprises glass.

In a preferred embodiment, the capture probes are arranged in clusters, and wherein the clusters on the substrate are obtained by exclusion amplification.

In yet another preferred embodiment, the nanowell array is characterized by repeating arrangement of nanowells, preferably such that the relative arrangement of nanowells at one part of the array is the same as the relative arrangement of nanowells at at least one other part of the array.

In a further preferred embodiment of the invention, the substrate is a visibly marked nanowell substrate, and preferably comprises at least one, preferably at least two or more, visible markings of a known location on the substrate. The markings allow for an alignment of an optical image taken in step (c) with the positioning of the locations of the clusters of capture probes on the substrate.

Preferably, the method further comprises subsequently to step (b) a step (b') of fixating the tissue sample, preferably wherein the fixation comprises the use of an alcohol as fixating agent. More preferably, the fixation is performed in a alcohol-based fixative, such as an Methanol as disclosed herein elsewhere.

In some embodiments of the invention, the method is comprising a step (f) of correlating the location of the corresponding location of the capture probes on the substrate with a corresponding location within the tissue sample, thus identifying the spatial location of RNA (e.g., gene) expression in the sample.

In another preferred embodiment, the patterned nanowell substrate comprises a regular engraved pattern of the visible markings, preferably wherein the regular engraved pattern is optically visible through a tissue sample placed on top of the patterned nanowell substrate.

In some embodiments, the engraved areas on the nanowell substrate are free of capture probes.

Preferably, visible markings are characterized by a lack of capture probes on the support. More preferably, the visible markings allow for aligning optical imaging and transcriptomic data. The visible markings are in context of the invention used as fiducial markers, therefore refer to either a virtual (such as computer-generated) or physical objects used in the field of view of an imaging system which appears in the image produced, for use as a point of reference or a measure. It may be either something placed into or onto the imaging subject, or a mark or set of marks in the reticle of an optical instrument. In the present case the visible markings can be engraved structures on the substrate, and are used to align the optical image.

Preferably, the method comprises that the substrate is placed within a cultivation and/or reaction chamber to allow for a treatment in accordance with the invention, such as nucleic acid amplification, tissue sample fixation etc.

In accordance with the herein described invention, the cultivation and/or reaction chamber allows for delivering reagents to the chamber and removal of reagents, preferably wherein the chamber is a well on a multi-well reaction device, such as a multi well plate.

Preferably, the substrate for use of the invention has a dimension of 1 to 1000 mm², preferably of 5 to 600 mm², more preferably of about 500 to 600 mm². For example, in certain preferred embodiments, the substrate of the invention is preferred wherein substrates fits into a well of a multi well plate, and may range from about 5 mm² 50 mm².

In accordance with the invention the nucleic acid can be selected from any nucleic acid such as DNA or RNA, but in a preferred embodiment is RNA.

A tissue sample in accordance with the invention is a cellular tissue sample. The Tissue may be intact or the cells of the tissue are separated.

In **a second aspect**, the invention pertains to a method for spatial detection of RNA in a tissue sample, comprising:
(a) providing the substrate of any of the preceding claims;
(b) contacting the substrate with a tissue sample and allowing RNA molecules of the tissue sample to bind to the capture domain of the capture probes;
(c) fixating and/or permeabilizing the tissue sample and thereby allowing RNA molecules of the fixed and permeabilized tissue sample to bind to the capture domain of the capture probes,
(d) generating cDNA molecules from the bound RNA molecules; and
(e) sequencing the cDNA molecules.

In **a third aspect**, the invention pertains to a kit of parts for use in the spatial transcriptomics, wherein the kit comprises at least a substrate as recited in any one of the preceding claims, and one or more buffers.

Preferably, the kit of the invention comprises a fixating solution comprising an alcohol. For example, the alcohol based fixation solution comprises methanol, and preferably does not comprise a cross linking agent, such as formaldehyde.

The kit may further comprise a permeabilization reagent, such as a pepsin-containing SSC buffer, such as a 2x SSC buffer. Preferably, the permeabilization agent has an acidic pH, preferably a pH of less than 4, more preferably less than 3, most preferably of about 2.5.

Furthermore, the kit further comprises a cutting guide, preferably a 3D printed cutting guide as described herein elsewhere, suitable for cutting a commercially available flow cell into smaller fragments.

A further aspect of the present invention then pertains to a cutting tool (10), comprising a rectangular base (11) flanked by two side walls (12) and (13) in longitudinal direction of the rectangular base (11), and wherein the side walls (12) and (13) are higher than the base support (11), the base (11) on the upper surface comprises a sliding caveat (20), comprising at least two sliding rail protrusions (21) in longitudinal direction, wherein cutting tool (10) further comprises at least one positioning beam (30), which attached to and connects the two side walls (12) and (13), wherein the at least one first positioning beam (30) comprises at least one positioning notch (31) on the lateral side of the positioning beam (30).

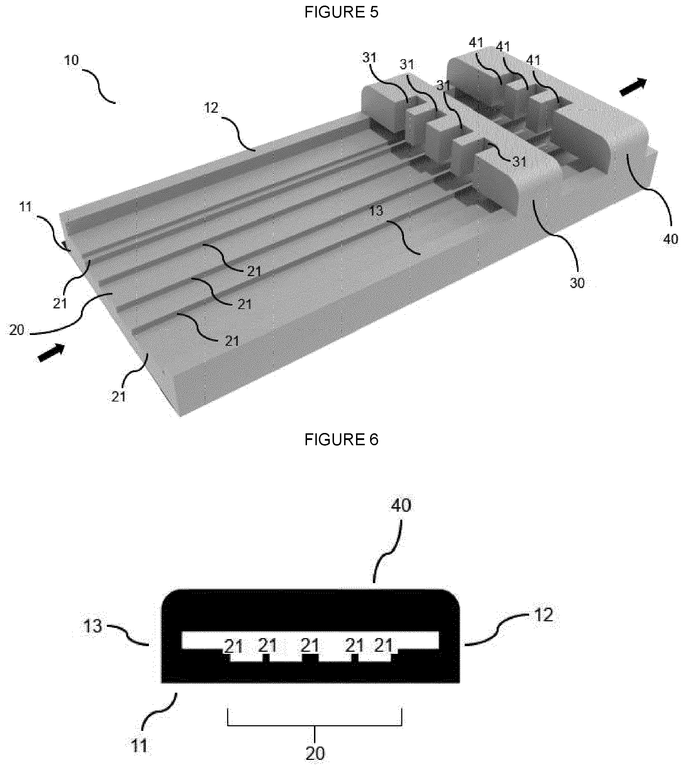

The cutting tool may be understood in certain embodiments as a "cutting guide" or "cutting device" and is required for fragmentation a for example commercially available flow cell after capture probe generation ("synthesis by sequencing") into smaller sections for use in the methods of the invention. A preferred embodiment of the cutting tool of the invention is provided in the figures 5 and 6.

In a preferred embodiment, the sliding caveat is in rectangular shape and dimensioned to receive a glass slide object, preferably a flow cell in the dimensions of 15.2 x 4.0 cm.

In another preferred embodiment, the sum of surfaces of the top area of all sliding rails (21) is less than the sum of the top area between the sliding rails (21) in the sliding caveat (20). Since the cutting guide operates by actively sliding a flow cell through the tool which fixes a glass cutter at a certain pre-determined position, the feature reduces sliding friction and thereby reduces incomplete cutting, or cutting errors during the process.

More preferably, the cutting tool comprises at least 3, 4 or 5 sliding rails (21).

In other embodiments the cutting tool is comprising at least a first positioning beam (30) and at least one second positioning beam (40), wherein the first positioning beam (30) and the at least one second positioning beam (40) are spaced from each other in longitudinal direction.

It is preferable in some embodiments that the at least one positioning notch (31) within the first positioning beam (30) is located on the same lateral side as the at least one positioning beam (41) within the at least one second positioning beam (40).

In some embodiments, the at least one positioning notch (31) and the at least one positioning beam (41) are in an offset longitudinal position.

The cutting tool (10) of the invention in some embodiments that are preferred is characterized in that the at least one first positioning beam (30) comprises at least two, preferably at least three, more preferably at least four, most preferably at least six positioning notches (31).The cutting tool (10) of any one of the preceding claims, wherein the at least one second positioning beam (40) comprises at least two, preferably at least three, more preferably at least four, most preferably at least six positioning notches (41).

The cutting tool (10) of the invention is preferred, wherein the at least one positioning notch is positioned such, that when a flow cell is positioned within the sliding caveat, the positioning of a glass cutter allows for cutting the flow cell in longitudinal direction between two channels (or lanes) of the flow cell.

Further, the invention provides a cutting tool (10), wherein the at least one positioning notch is positioned such, that when a flow cell is positioned within the sliding caveat, the positioning of a glass cutter allows for cutting the flow cell in longitudinal direction within one channel or lanes of the flow cell and thereby halving the flow cell channel or lane.

In another aspect there is provided a use of a cutting tool according to the invention, for cutting a flow cell. The cutting tool allows to fragment a for example commercially available flow cell into smaller sections that are useful for certain applications. Preferably, the sections are used for methods of the invention.

Furthermore, the invention provides a method for cutting a glass flow cell, comprising the steps of placing a flow cell into the sliding caveat, placing a glass cutter on top of the flow cell and within one of the positioning notches, and cutting the flow cell by pulling the flow cell along the longitudinal direction on top of the sliding caveat and thereby cutting the flow cell in longitudinal direction. Preferably the method comprises repeating the steps for cutting with the same flow cell at least once with the glass cutter placed in a at least one other positioning notch.

The cutting tool of the invention is preferably used to produce the support from a cut flow cell.

A cutting tool of the invention is used for a guided fragmentation of a flow cell.

The invention in one additional aspect pertains to a use of a cutting tool for a controlled cutting of a flow cell into smaller supports.

As used herein, the term "flow cell" is intended to mean a chamber having a surface across which one or more fluid reagents can be flowed and to which nucleic acids can bind and seed the surface (s) of the flow cell. Generally, a flow cell will have an ingress opening and an egress opening to facilitate flow of fluid. Examples of flow cells and related fluidic systems and detection platforms that can be readily used in the methods of the present disclosure are described, for example, in Bentley et al. , Nature 456:53-59 (2008) , WO 04/018497; U.S. Pat. No. 7, 057, 026; WO 91/06678; WO 07/123744; U.S. Pat. No. 7,329, 492; U.S. Pat. No. 7,211, 414; U.S. Pat. No. 7,315, 019; U.S. Pat. No. 7, 405,281, and US 2008/0108082, each of which is incorporated herein by reference. [0035] In a certain embodiment, a flow cell includes a solid support having a surface on which nucleic acids can bind. In some embodiments, the surface contains a lawn of capture agents that can bind to released polynucleotides from beads. In some examples, the surface is a patterned surface. A "patterned surface" refers to an arrangement (such as an array) of different regions (such as amplification sites) in or on an exposed surface of a solid support. For example, one or more of the regions can be features where one or more capture agents are present. The features can be separated by interstitial regions where the capture agents are not present. In some embodiments, the pattern can be an x-y format of features that are in rows and columns. In some embodiments, the pattern can be a repeating arrangement of features and/or interstitial regions. In some examples, the pattern can be a random arrangement of features and/or interstitial regions. In some embodiments, the surface is a patterned surface that contains an array of wells with capture agents that bind to released polynucleotides from beads, with interstitial regions between the wells that lack the capture agents. In several cases the substrates are exemplified in these references for applications that use beads in the wells. The patterned flow cells can be used with the compositions, methods and kits of the present disclosure.

A patterned flow cell comprises an array of features, such as microwells or nanowells, on glass, silicon, plastic or other suitable solid supports. The array of features can comprise capture agents which, in one embodiment, are covalently-linked to a gel on the surface of the well, such as poly (N- (5-azidoacetamidylpentyl) acrylamide) (PAZAM, see, for example, U.S. Pub. No. 2013/184796, WO 2016/066586, and WO 2015/002813, each of which is incorporated by reference herein in its entirety). The lifetime of the flow cell can be extended by covalently linking the polymer to the features on the flow cell surface. However, in many examples the gel need not be covalently linked to the features on the flow cell surface. For example, silane free acrylamide (SFA, see, for example, U.S. Pat. No. 8,563, 477, which is incorporated by reference herein in its entirety) can be used as the gel material. SFA is not covalently attached to the features on the flow cell surface. Examples of flow cells with patterned surfaces that can be used in the methods set forth herein are described in U.S. Pat. Nos. 8,778,848, 8,778, 849 and 9, 079, 148, and U.S. Pub. No. 2014/0243224, each of which is incorporated by reference herein in its entirety. The array of features on the surface of flow cells can be used to capture a single nucleic acid template molecule to seed subsequent formation of a homogenous colony, for example, via bridge amplification. Such patterned arrays are particularly useful for nucleic acid sequencing applications.

Preferred flow cells that are commercially available and can be used in accordance with the invention, are NextSeq Flow cell P1,P2,P3 (Illumina, 2023) available in the following kit: NextSeq 1000/2000 P1 Reagents (20074933, 20050264, 20075294); NextSeq 1000/2000 P2 Reagents v3 (20046811, 20046812, 20046813, 20075295); NextSeq 2000 P3 Reagents (20046810, 20040559, 20040560, 20040561); NovaSeq 6000 Flow cell SP, S1, S2, S4 (Illumina, 2023) available in the following kit: NovaSeq 6000 SP Reagent Kit v1.5 (20028401, 20040719, 20028400, 20028402); NovaSeq 6000 S1 Reagent Kit v1.5 (20028319, 20028318, 20028317); NovaSeq 6000 S2 Reagent Kit v1.5 (20028316, 20028315, 20028314); NovaSeq 6000 S4 Reagent Kit v1.5 (20044417, 20028313, 20028312).

The cutting tool of the invention allows for cutting in particular a nanowell area on a patterned nanowell flow cell into smaller patterned nanowell supports in accordance with the invention.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

### BRIEF DESCRIPTION OF THE FIGURES AND REFERENCE SIGNS

The figures show:

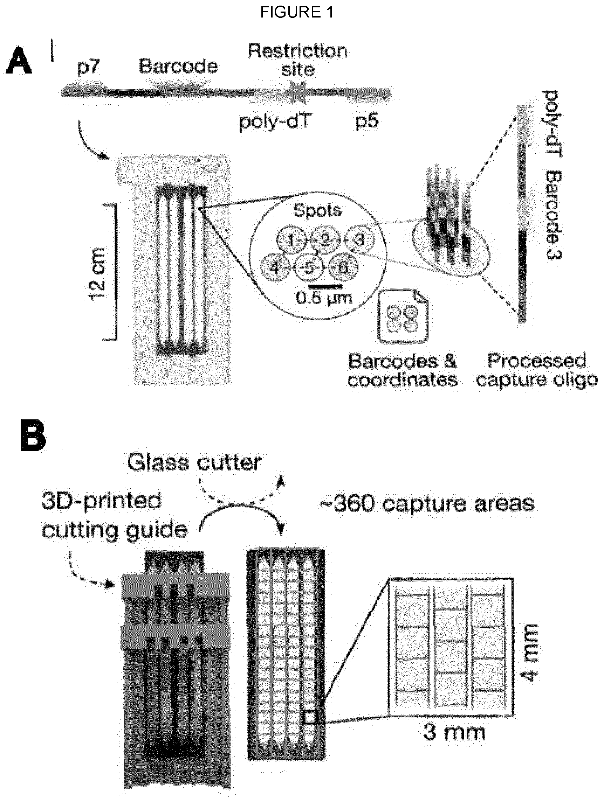

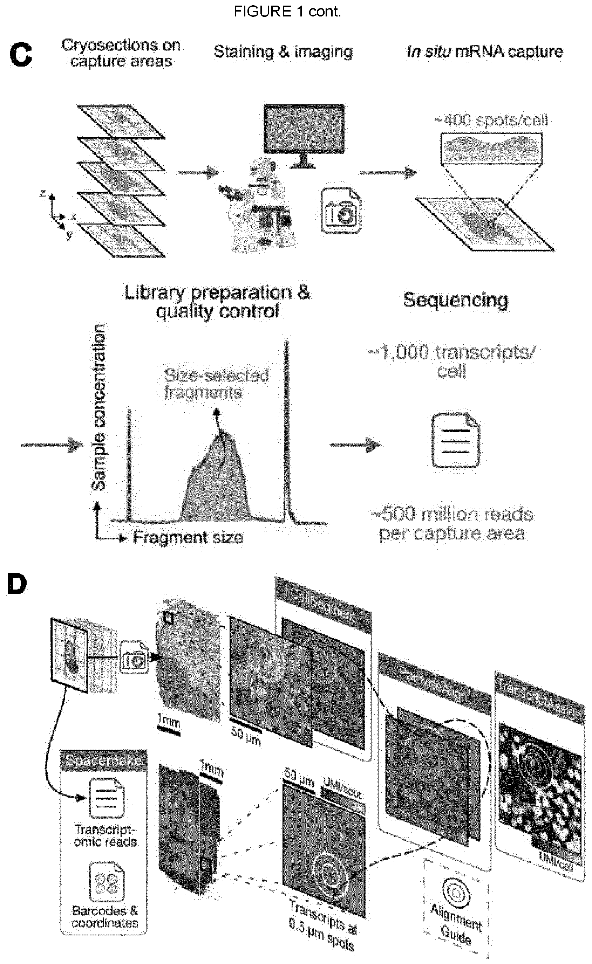

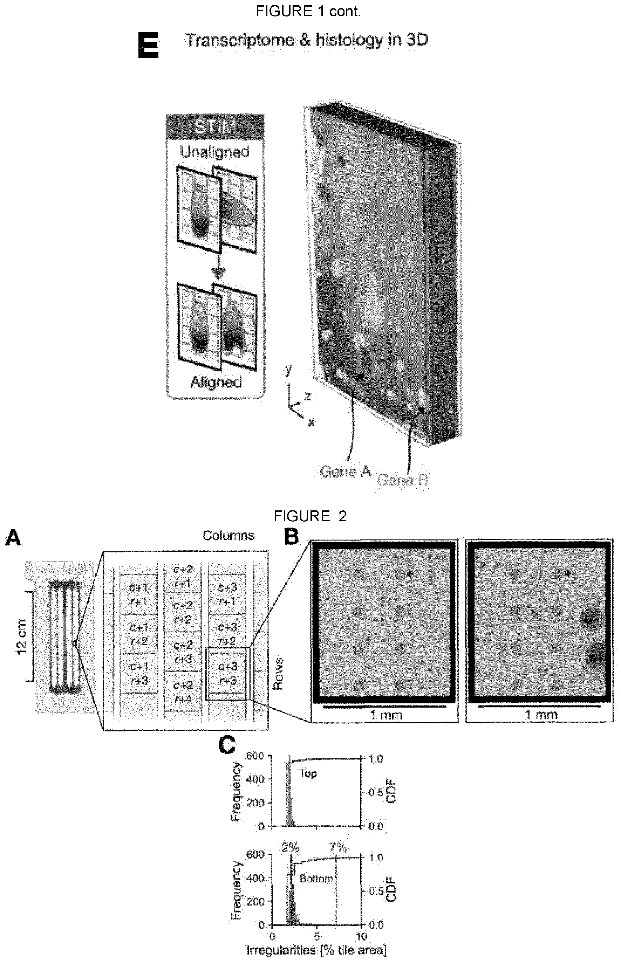

**Figure 1**: Open-ST workflow for high-resolution spatial transcriptomics of segmented single-cells in 2D or 3D (A) Sequencing of designed oligos in patterned Illumina flow cells (here: NovaSeq6000 S4) allows barcode registration in regularly spaced spots at 0.5 µm center-to-center distance (left) High-density spots with clonal oligonucleotides are processed to end with a poly-dT stretch, for capturing poly-adenylated RNA (right). (B) The opened processed flow cell is placed on our custom 3D-printable device that allows guided cutting to produce ~360 capture areas of size 3x4 mm 2 and at -45E each (Methods). The capture area is composed of ~1 mm2 tiles across 3 columns. Between columns, there is a distance of 55 µm that lacks registered barcodes (Figure 2B). p5/p7: Illumina adapters. Flow cell image courtesy of Illumina, Inc. (C) Transcriptomic and H&E imaging data are generated from the same fresh-frozen tissue section. Optimized RNA capture conditions and a single-amplification library preparation result in high library complexity (Methods). FU, fluorescent units. (D) Tissue morphological information (imaging) is integrated with spatial transcriptomics data from single sections with our open-source openst Python package, including code for automatic cell segmentation, pairwise alignment of modalities, and quantification of transcripts (unique molecular identifiers, UMI) to segmented cells. (E) Serial sections can be used for three-dimensional reconstruction of tissue histology and transcriptome, using STIM. Imaging and transcriptomics data can be visualized and interrogated as a 3D virtual tissue block (right). The smoothed, volumetric rendering of two genes (A: S100A7; B: FDCSP) is shown for illustration purposes.

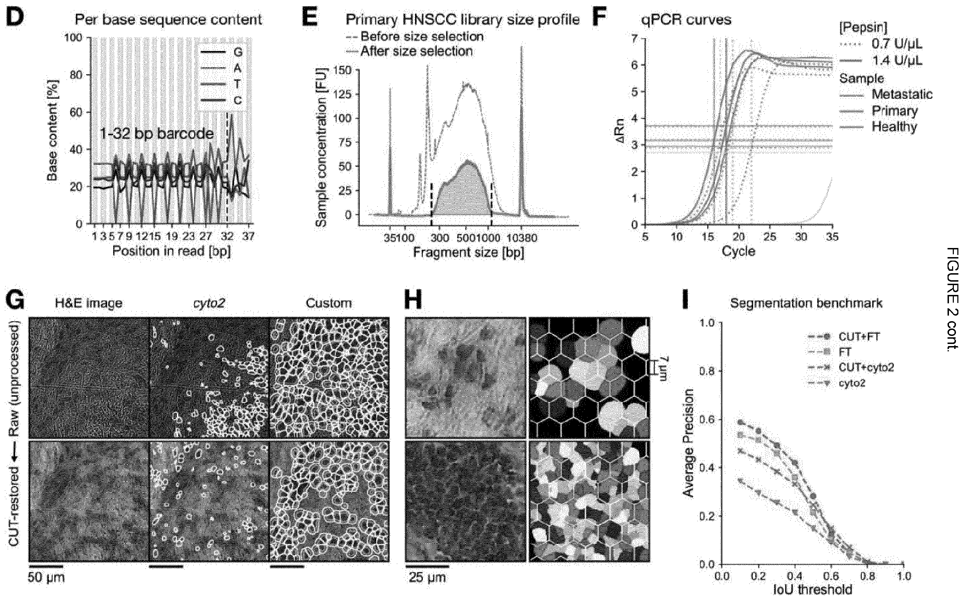

**Figure 2**: Open-ST workflow quality control and image processing, related to Figure 1 (A) A NovaSeq S4 flow cell consists of 4 lanes with a top and bottom surface, each with 6 columns (c) and 78 rows (r) per lane, totalling 3744 tiles. Tiles are discrete sections of the flow cell imaged during sequencing. Distance between tiles is 55.5 µm in the x- and 5.3 µm in the y-axis. (B) Representative example of tiles with average number of irregularities (left) and higher number of irregularities (right). Blue star: space without spots due to fiducial markers; red arrowhead: imperfections that may result from flow cell manufacturing errors (small black dots), bubbles during sequencing (medium black areas), or dust obstructing the imaging (large black areas). (C) Frequency of irregularities (>2 µm) on the flow cell fc_1 from the 1st sequencing. The top flow cell surface contains fewer irregularities (CDF, cumulative density function). (D) Per base sequence content of 1st sequencing of fc_1. Bases 1 to 32 correspond to the barcodes, with drops in "A" or "T" at expected sites in sequence (B or V in IUPAC nucleotide code). (E) Automated electrophoresis profiles of the primary HNSCC library before and after size-selection. Fluorescent units (FU) relate to bioanalyzer input and not total sample concentration. Peaks at 35 and 10380 bp are the upper and lower markers. (F) Comparison of tissue permeabilization conditions via qPCR assay. Example data with two pepsin concentrations tested per tissue type: permeabilization with higher concentration captured more mRNA. Determination of cycling number from qPCR assay is described in the methods section "PCR cycling number assessment". (G) Image restoration and segmentation pipeline: raw images after stitching (upper), and restored images after applying a Contrastive Unpaired Translation model (Methods). Left: H&E images used as input; middle: segmentation masks generated by Cellpose 2.0; right: segmentation masks via our fine-tuned version. (H) Robustness of segmentation for different cell densities; two regions with -65% (upper) and -99% density (bottom) shown for the metastatic lymph node. CUT-restored images (left) were segmented with our fine-tuned model (right). Each segmented cell (nucleus with a 3.5 µm extension) is depicted with a different color. A regular lattice of hexagons (7 µm side) is overlaid onto the segmentation masks, illustrating the difference between data meshing and segmentation. (I) Benchmark of the segmentation models, measured as the average precision of the restoration+segmentation models at different intersection over union (IoU) thresholds.

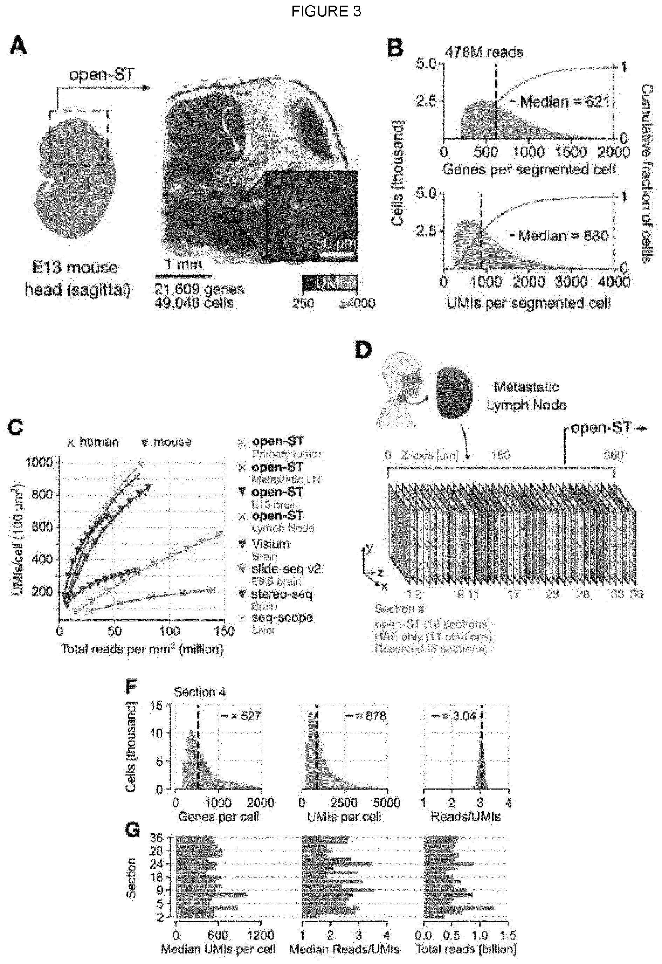

**Figure 3**: Open-ST robustly captures the whole transcriptome with high efficiency (A) Spatial distribution of UMI counts per segmented cell in an open-ST processed E13 mouse head sagittal section. (B) Distribution of genes and UMIs per segmented cell; sample as in (A). (C) Open-ST outperforms competing sequencing-based spatial transcriptomic methods, exhibiting the highest transcript capture per pseudo-cell (UMIs/100µm²) for the same number of sequencing reads. (D) Experimental design for processing a human metastatic lymph node with open-ST. 10µm thick sections were processed and profiled with open-ST, only H&E stained, or reserved for validation. (E) Aligned transcriptomic and imaging data; 3D-rendering of transcript capture coloured by UMI counts is overlaid on a virtual tissue block. (F) Distributions of UMIs and gene counts per segmented cell for section 4, which was sequenced the deepest due to its low reads/UMI ratio (G) Total reads, median UMIs and median reads/UMI per segmented cell across all 19 sections show consistently high transcript capture and low ratio of sequencing reads to UMls captured.

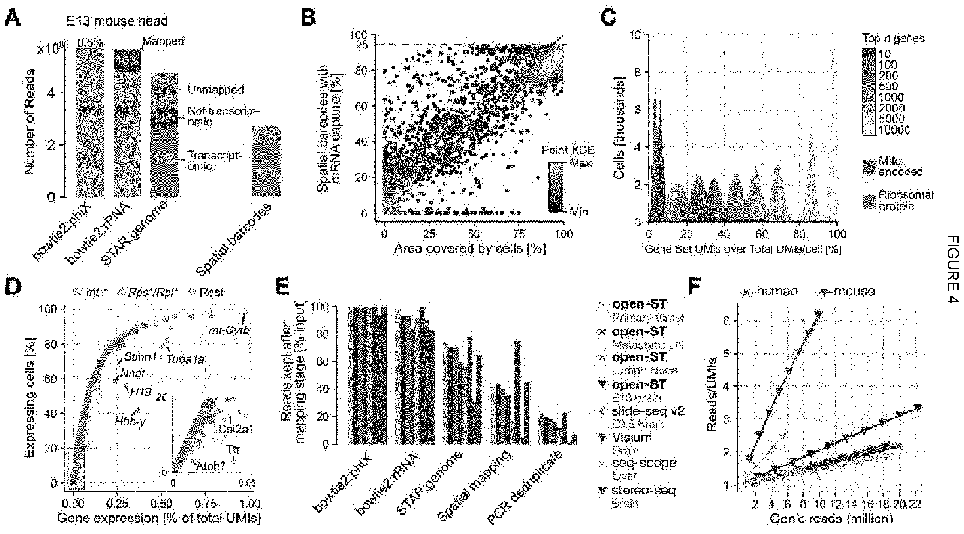

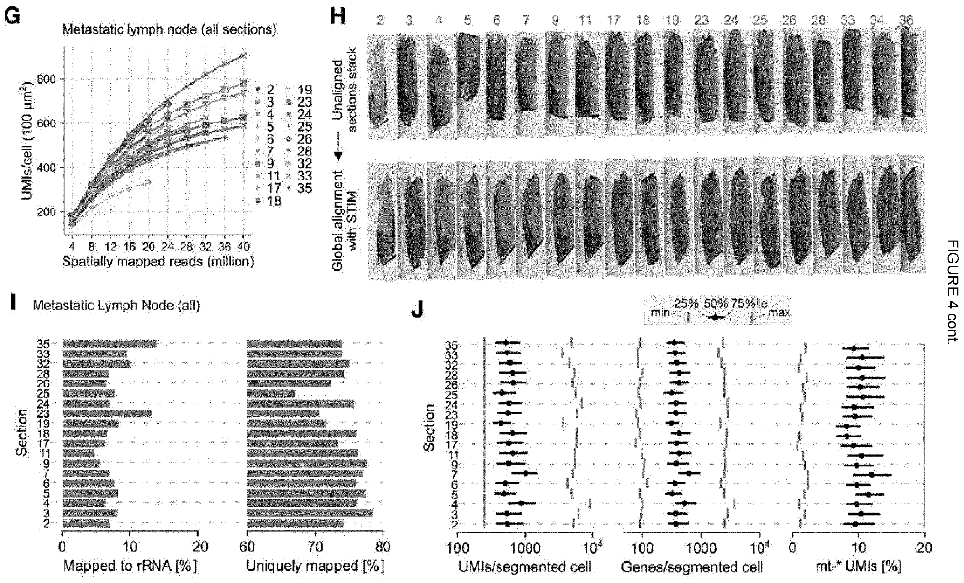

**Figure 4**: Reproducibility and performance of open-ST, related to Figure 3; (A-C) (A) (A) Stacked barplot of input reads filtered out during two-stage alignment against PhiX and mouse rRNA, summarized STAR mapping statistics from the remaining reads, and percentage of transcriptomic reads with matching spatial barcodes, for E13 mouse head dataset. Not transcriptomic: reads with ambiguous and intergenic mapping; transcriptomic: reads mapping to UTR, intron and coding loci. (B) Percentage of spatial barcodes with captured transcripts relative to the area covered by cellsat non-overlapping -5,000 µm² square regions. Diagonal dashed line: y=x; horizontal dashed line: ymax across the dataset. Coloring shows point density. (C) Percentage of total unique molecular identifiers (UMI) per segmented cell accounting for several gene sets: top n genes by total UMI, mitochondrially-encoded transcripts (mt-*), and ribosomal protein transcripts (Rps*/Rpl*). (D) Transcriptomic information measured as the percentage of cells expressing a gene, over the total UMIs of that gene across cells. (E) Ratio of sequencing reads to UMIs captured for open-ST and competing sequencing-based methods. (F) Library complexity measured as reads/UMIs ratio over number of genic reads across samples. (G) Sequencing investment measured as UMIs/cell per 100µm² over spatially mapped reads for all 19 sections of the metastatic lymph node sample. (H) Stacked 3D rendering of H&E images before (top) and after global alignment with STIM (bottom). (I) Left: % reads mapping to rRNA after excluding PhiX-mapping reads; right: % reads uniquely mapping to the genome after excluding rRNA-mapping reads. Data shown across all 19 sections of the metastatic lymph node. (J) Distribution of UMIs and genes captured per segmented cell, as well as % UMIs mapping to the mitochondrial genome, across all 19 sections of the metastatic lymph node. In C and D, cells with less than 250 or greater than 10,000 UMI, and more than 10% mitochondrial counts, are removed; genes are filtered out when present in less than 10 cells. In H, I and J, cells with less than 250 or greater than 10,000 UMI, and more than 20% mitochondrial counts, are removed.
**Figure 5****: shows a cutting tool (10) in accordance with the invention.**
**Figure 6****: shows a front view of the cutting tool (10) of the invention.**

### Reference signs:

| | | |
|---|---|---|
| • | 10 | Cutting Tool |
| • | 11 | Base |
| • | 12 | first side wall |
| • | 13 | second side wall |
| • | 20 | sliding caveat |
| • | 21 | Sliding rail |
| • | 30 | first positioning beam |
| • | 31 | positioning notch |
| • | 40 | second positioning beam |
| • | 41 | positioning notch |

### EXAM PLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Process of the Invention ("Open-ST")

Figure 1 schematically represents the experimental protocol in a preferred implementation of the invention (1A, 1B) as well as the computational workflow for spatial single-cell analysis in 2D and 3D (1C, 1D). Illumina's sequencing-by-synthesis technology is leveraged to generate a surface covered in barcoded oligonucleotide clusters, later processed to be available for polyadenylated transcript capture. Tightly packed clusters (ie. capture spots) with a unique 32-nucleotide spatial barcode are created by bridge amplification, each associated with a x-y coordinate on the physical array. The inventors use the NovaSeq6000 technology yielding higher-density patterned capture spots (≤ 400 spots vs Seq-scope's Miseq 150 spots/100 µm²) in a larger capture area. The capture spots are regularly arranged with an average center-to-center distance of 0.6 µm, making efficient use of the surface area and contributing to the high resolution.

Following the sequencing of designed capture oligos and processing to allow polyA transcript capture, the flow cell is opened and cut into small capture areas (which constitute in certain embodiments the substrates of the present invention), whilst preventing surface scratches: The dimension of the capture area can be chosen based on the experimental design, with a maximum size of 7 x 80 mm as limited by the sequenced area of the flow cell. The substrates of the invention allow for analysis of tissue morphology with a high-quality H&E staining and localized capture of RNA transcripts from the same cryo-section (Figure 1B). Permeabilization enzyme and hybridization buffer are combined in one solution to promote the simultaneous tissue opening (RNA release) and RNA capture. A qPCR assay is used to determine optimal permeabilization conditions for maximum mRNA capture. Implementation of a further qPCR-based quality control during library preparation allows us to determine optimal cycling number avoiding library over- or under-amplification.

Additionally, the one-step library amplification prevents PCR amplification bias or sample losses due to bead purification in between PCR reactions. The lack of specialized equipment required facilitates transferability between laboratories.

The inventive analysis pipeline combines morphological features with RNA expression, offers the possibility to analyze the data for segmented cells and combine serial slide data (Figure 1D).

The open-source Spacemake pipeline is used to process the transcriptomic reads and map them in space using the barcode coordinate file from the first barcode sequencing (Sztanka-Toth et al., 2022). Then, imaging- and sequencing data are combined via pairwise alignment with an accuracy of 0.5 -1 µm, using the inventive circular marks on the flow cell as alignment guides. These guides, visible in both the imaging and spatial transcriptome modalities, can be automatically detected for unsupervised alignment (a detailed description of this procedure is provided in example 4). The H&E raw images are automatically preprocessed (Figure 2G) and then segmented into single cells using a fine-tuned Cellpose model (Figure 2H). Accordingly, data can be analyzed on a single cell level and related to its associated morphology.

The method of the invention with its low cost per sample and the integrated pipeline is ideal to be used for a 3D reconstruction of any sample. The inventors apply it for the 3D reconstruction of a metastatic lymph node (Figure 1E). The objects of serial sections were aligned using STIM (Preibisch, Karaiskos, and Rajewsky 2022). The inventors can interrogate both the imaging and transcriptomics data from any perspective in the 3D reconstruction.

### Example 2: Method of the invention ("Open-ST") efficiently captures the whole transcriptome in space

The inventors demonstrated the applicability of the inventive method to various sample types: embryonic mouse head adult mouse hippocampus, human primary tumor (HNSCC) and the patient-matched normal (LN) and metastatic lymph nodes (mLN) (Figure 3).

High-quality H&E staining allowed nuclei segmentation of all datasets via a deep learning image processing pipeline; subsequently, radial extension of nuclei boundaries adds cytoplasmic context, yielding approximate cell boundaries (Figure 2G, 2H). The segmentation and alignment protocol unbiasedly adapts to tissues with heterogeneous cell sizes and densities, and automatically excludes tissue areas without cells from downstream analyses (Figure 2I).

All samples had a high percentage of transcripts mapping to the genome (65-78%) and only a small percentage of input reads consisted of ribosomal RNA (Figure 4A, 4G, 4I). For the E13 mouse and metastatic lymph node 72% and 82% of reads uniquely mapping to untranslated regions, intronic or coding sequences could be assigned to a spatial barcode from the first sequencing run. Spatially mapped transcriptomic reads represent 34% (161/478 million) and 42% (523/1 259 million) of raw reads from the second sequencing (Figure 4A, 4G). This constitutes the amount of information useful in downstream analyses. The percentage of barcode reads from the 1st sequencing detected in the transcriptome library relates to tissue coverage and cell density (Figure 4B).

In a sagittal E13 mouse brain section with 49 048 segmented cells sequenced at a depth of 478 million reads, the inventors captured a median of 621 genes and 880 UMIs, with 42% of the cells containing over 1 000 transcripts (Figure 3A, 3B). The inventors capture a high diversity of genes, with the top 200 genes accounting on average for 56% (median of 53%) of the UMIs captured per segmented cell (Figure 4C).

Library complexity of open-ST data was compared to existing sequencing-based ST methods, by calculating UMI counts per pseudo-cell (100 µm²) relative to sequencing depth (Figure 3C and 4E-4F). Open-ST outperforms all leading competitors in capture efficiency. Unique transcript-capture was comparable between the samples despite their variability in cellular composition and RNA quality (RNA integrity number of 6.7, 8.3 and 8.9 for mLN, HNSCC and E13 mouse head, respectively). Furthermore, the down-sampling curve extended to 80 million reads per 1 mm² indicates that sequencing saturation has not been reached for these samples meaning more information can be gained by sequencing deeper (Figure 4E). Moreover, the inventors compared the reads to UMI ratio of the normalized data down sampled for sequencing depth.

### Example 3: The method of the invention ("Open-ST") is highly reproducible across tissue sections

A large-scale experiment on the mLN serves as a proof-of-principle that open-ST can reproducibly capture the transcriptome across many sections in a complex tissue of clinical interest. In total the inventors obtained gene expression profiles of over 1 million cells across 19 serial sections with a median capture of 313 to 624 genes and 438 to 1008 UMls per segmented cell (Figure 4J). Smooth isosurface renderings of the spatially mapped UMI counts indicate the reproducible capture efficiency with a visible transcript enrichment in the tumor compartment, likely due to active proliferation of these cells (Figure 3D). The reads/UMI ratio per segmented cell is low and comparable between sections, supporting the reliability of the method and the low sequencing investment required (Figure 3E). Moreover, the percentage of reads mapping to rRNA (<15%) and transcripts uniquely mapping to the genome (>60%) are consistent between sections (Figure 4I). Reproducible detection of gene expression is supported by the high correlation of the pseudo-bulk expression profiles (r >0.95) (Figure 4L).

In summary, the method of the invention robustly captures the whole transcriptome in various tissues and across many sections of the same tissue, with low sequencing investment needed, supporting its utility for high-throughput studies such as 3D-transcriptome reconstruction or the analysis of patient cohorts.

### Example 4: Preprocessing and cell segmentation of H&E images

Tile-scan images of H&E-stained tissue sections were stitched together using the Grid/Collection stitching plugin included in Fiji 1.53t (Schindelin et al. 2012), generating a composite image of the entire section. RGB color tile-scans were converted to a HSV (Hue/Saturation/Value) image; then, the saturation channel was blurred with a Gaussian filter, and binarized with Otsu thresholding. This delivers a mask used to isolate the tissue from the background, mostly consisting of the flow cell piece. Optionally, tile-scans were further preprocessed by style transfer with a custom Contrastive Unpaired Translation (CUT) model (Park et al. 2020), trained with default parameters on unpaired 512x512 pixel patches from tile-scans imaged with high noise/variance (source style), and low noise/variance (destination style). This model was specifically applied to the metastatic lymph node H&E imaging data to equalize the style between sections and remove artifacts due to low aperture size during acquisition.

Then, nuclei were segmented with cell pose 2.2 (Stringer et al., 2021; Pachitariu and Stringer, 2022), upon fine-tuning the cyto2 model on pairs of H&E images from the metastatic lymph node sample (section 4) and manually refined segmentation masks from the same pretrained model. Segmentation of all images was performed with --diameter 20 (-7 µm),-flow_threshold 2, and --cellprob_threshold -1, unless otherwise stated. Segmented nuclei were extended 10 pixels (-3.45 µm), radially and in a non-overlapping manner, using the function segmentation.expand_labels from scikit-image (v0.19.3) (van der Walt et al., 2014). In samples with adipocytes (e.g., healthy human lymph node sample), a second round of segmentation with this fine-tuned cell pose model was carried out, with --diameter parameter set to 100 pixels (-34.5 µm). Segmentation masks were similarly extended 50 pixels (-17.25 µm) radially. To keep all small and large segmented cells, two rounds of segmentation were performed in the same image. Then, the two segmentation masks (one with and other without adipocytes) were merged by removing segmented cells from the mask with small diameter using the segmentation with large diameter as a negative binary mask. Then, the filtered small-nuclei mask and adipocyte mask were combined via AND operation.

A two-step protocol was designed to align spatial transcriptomics data to tile scans of tissue staining (H&E in this study) from the same section, such that capture spot data can be aggregated into (segmented) single cells, instead of using arbitrary grids. This protocol relies on the generation of pseudoimages from the spatial transcriptomics data (see "Generation of open-ST pseudoimages"), using libraries from scikit-image. First, rescaled H&E images (-7 µm/pixel) were coarsely aligned to low-resolution (-7 µm/pixel) pseudoimages of ST data via the pre-trained Detector-Free Local Feature Matching with Transformers (LoFTR) outdoor model, from kornia (v0.7.0). A robust transformation model was estimated with Random Sample Consensus (RANSAC). For a more precise alignment (-1 µm error), fine registration was performed, leveraging feature matching on H&E images and pseudoimages with higher resolution (-1.5 µm/pixel). Fiducial markers visible on both modalities were detected on the full-resolution images (0.345 and 0.5 µm/pixel) and appended to the features used for fine registration. The detection of fiducial marks was carried out automatically with custom YOLO-based object detection models. Likewise, the LoFTR-detected features and YOLO-detected marks were matched across modalities via brute-force and nearest neighbors matching, followed by RANSAC estimation of the transformation model. Since pairwise alignment is performed between an H&E image and ST data from the same section, rigid models were used to transform ST coordinates into H&E image space, as no distortions were expected. Finally, the napari graphical environment was used for visual assessment and manual refinement of the alignment, if necessary.

Finally, spatial cell-by-gene expression matrices were created by aggregating the initial NxG matrix (N, capture spots; G genes) into a MxG matrix (M, segmented cells; G, genes), where the mapping of N to M takes place via the segmentation mask. That is, for all segmented regions (see "Stitching and cell segmentation of H&E-stained images"), spots falling within the spatial coordinates of a segmented region are aggregated into a single "segmented cell" with identifier equal to the cell mask label.

The generation of pseudoimages was tailored to distinct visualization needs, encompassing two methodologies. On the one hand, for aggregated cell-level depictions in 2D, the pl.spatial function from the scanpy v1.9.3 package was used. On the other hand, higher-resolution visualizations focusing on individual transcripts (typically, at -100x100 µm zoom) relied on local 2D Kernel Density Estimation (KDE) of UMI-scaled spatial coordinates, for any chosen transcript. The KDE was performed with a bandwidth of ~1 µm. For visualization, the KDE is treated as image data visualized with the micro show function from microfilm v0.2.1 package. Intensity limits were adjusted from the 5th to the 95th percentile. These renderings are useful to visualize and quantify transcript density in space, rather than the sparser raw counts. Alternatively, raw UMI counts can be displayed as an image by producing an empty canvas defined by re-centered coordinate bounds, mapping coordinates onto the canvas, and aggregating UMIs within pixel bins. These resultant images resemble conventional pixel-based grids and can be readily subjected to standard image processing techniques.

## Claims

1. **A method of spatial transcriptomics on a substrate, the method comprising the steps of:**
(a) providing a substrate, wherein the substrate comprises at least two spatially separated capture probes immobilized thereon, and wherein the sequence of each of the at least two spatially separated capture probes are associated with its spatial localization on the substrate (spatial barcode);
(b) contacting the substrate with a tissue sample and allowing RNA molecules in the tissue sample to bind to the capture probes;
(c) imaging the tissue sample while the sample is bound to the substrate;
(d) generating cDNA molecules from the RNA molecules bound to the capture probes;
(e) determining the sequence of the spatial barcode for the cDNA molecules and correlating this sequence with the location of a corresponding cluster on the substrate (e.g., cluster of capture probes containing the corresponding spatial barcode);
***characterized in that**,*
the substrate comprises a patterned nanowell array.

2. The method of claim 1, wherein the method further comprises subsequently to step (b) a step (b') of fixating the tissue sample, preferably wherein the fixation comprises the use of an alcohol as fixating agent.

3. The method of claim 2, wherein the permeabilization is performed in a SSC buffer, such as an about 2x SSC buffer.

4. The method of any one of claims 1 to 3, further comprising a step
(f) correlating the location of the corresponding location of the capture probes on the substrate with a corresponding location within the tissue sample, thus identifying the spatial location of RNA (e.g., gene) expression in the sample.

5. The method of any one of claims 1 to 4, wherein the patterned nanowell substrate comprises a regular engraved pattern, preferably wherein the regular engraved pattern is optically visible through a tissue sample placed on top of the patterned nanowell substrate.

6. The method of any one of claims 1 to 5, wherein the capture probes are arranged in clusters, and wherein the clusters on the substrate are obtained by exclusion amplification.

7. The method of claim 6, wherein the nanowell array is **characterized by** repeating arrangement of nanowells, preferably such that the relative arrangement of nanowells at one part of the array is the same as the relative arrangement of nanowells at least one other part of the array.

8. The method of any one of the preceding claims, wherein the capture probe comprises a barcode unique for a spatial localization of a cluster of probes.

9. The method of any of the preceding claims, wherein each capture probe further comprises a cleavage domain, preferably wherein the cleavage domain comprises a binding site for a restriction endonuclease.

10. **A method for spatial detection of RNA in a tissue sample**, comprising:
(a) providing the substrate of any of the preceding claims;
(b) contacting the substrate with a tissue sample;
(c) fixating and permeabilizing the tissue sample and thereby allowing RNA molecules of the fixed and permeabilized tissue sample to bind to the capture domain of the capture probes,
(d) generating cDNA molecules from the bound RNA molecules; and
(e) sequencing the cDNA molecules.

11. The method of claim 10, wherein the fixation is performed using an alcohol as fixating agent, preferably, wherein the fixating agent does not comprise a cross-linking agent, such as formaldehyde.

12. **A cutting tool (10)**, comprising a rectangular base (11) flanked by two side walls (12) and (13) in longitudinal direction of the rectangular base (11), and wherein the side walls (12) and (13) are higher than the base support (11), the base (11) on the upper surface comprises a sliding caveat (20), comprising at least two sliding rail protrusions (21) in longitudinal direction, wherein cutting tool (10) further comprises at least one positioning beam (30), which attached to and connects the two side walls (12) and (13), wherein the at least one first positioning beam (30) comprises at least one positioning notch (31) on the lateral side of the positioning beam (30).

13. The cutting tool (10) according to claim 12, wherein sliding caveat is in rectangular shape and dimensioned to receive a glass slide object, preferably a flow cell in the dimensions of 15.2 x 4.0 cm.

14. **A kit of parts for use in the spatial transcriptomics**, wherein the kit comprises at least a substrate as recited in any one of the preceding claims, and one or more buffers, such as an alcohol-based fixation reagent.

15. The kit of parts of claim 14, comprising a cutting tool, or instructions for 3D printing a cutting tool according to claims 12 or 13.
